(19) Europäisches Patentamt
European Patent Office
Office européen des brevets

(11) Publication number: 0 149 331
A1

(12) # EUROPEAN PATENT APPLICATION

(21) Application number: 84308595.2

(22) Date of filing: 11.12.84

(51) Int. Cl.[4]: C 07 C 119/08

(30) Priority: 16.12.83 JP 237665/83

(43) Date of publication of application:
24.07.85 Bulletin 85/30

(84) Designated Contracting States:
DE FR GB

(71) Applicant: MITSUBISHI GAS CHEMICAL COMPANY, INC.
5-2, Marunouchi 2-chome Chiyoda-Ku
Tokyo(JP)

(72) Inventor: Sato, Katsuo
6-28 Fujimigaoka-1-chome
Ninomiyacho Naka-gun Kanagawa-ken(JP)

(72) Inventor: Kurokawa, Masahiro
95, Shinomiya
Hiratsuka-shi(JP)

(74) Representative: Baillie, Iain Cameron et al,
c/o Ladas & Parry Isartorplatz 5
D-8000 München 2(DE)

(54) **Process for producing methyleneimine compound.**

(57) A methyleneimine compound represented by the general formula:

$CH_2=N-CH_2-R$

wherein R is phenyl, alkyl-substituted phenyl or hydroxymethyl is produced by thermally decomposing a hexahydrotriazine compound represented by the general formula:

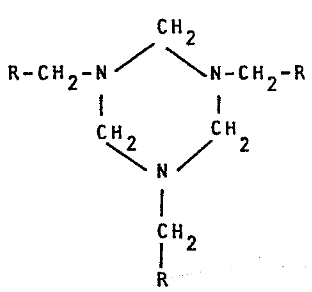

wherein R has the same meaning as defined above, at 130° to 220°C, preferably in the presence of a catalyst of an amine salt, a quaternary ammonium salt, or at least one of mono- to tri-valent metal salts of an organic acid or an inorganic acid.

EP 0 149 331 A1

# PROCESS FOR PRODUCING METHYLENEIMINE COMPOUND

## BACKGROUND OF THE INVENTION

This invention relates to a process for producing a methyleneimine compound, and more particularly to a process for producing a methyleneimine compound by thermal decomposition of 1,3,5-tribenzylhexahydrotriazine, 1,3,5-tris(alkyl-substituted benzyl)hexahydrotriazine, or 1,3,5-tris(2-hydroxyethyl)hexahydrotriazine (all of which will be hereinafter referred to merely as "hexahydrotriazine compound").

The desired compound of the present invention, that is, methyleneimine compound is a compound represented by the general formula:

$$CH_2=N-CH_2-R$$

wherein R is phenyl, alkyl-substituted phenyl, or hydroxymethyl, and only its presence has been so far confirmed, while its process has not been known.

## SUMMARY OF THE INVENTION

An object of the present invention is to provide a process for producing a methyleneimine compound by thermal decomposition of a hexahydrotriazine compound as a result of extensive studies of the process so far made by the present inventors.

The hexahydrotriazine compound for use in the

present invention as a starting material is a compound represented by the general formula:

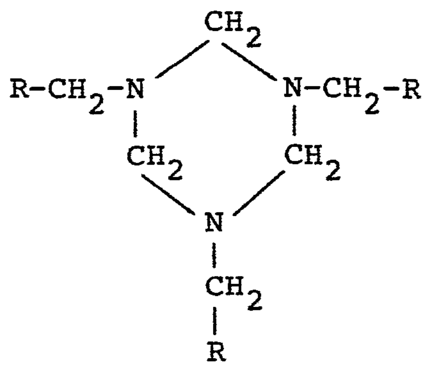

wherein R is phenyl, alkyl-substituted phenyl or hydroxymethyl.

The alkyl in the alkyl-substituted phenyl as R includes, for example, methyl, ethyl, propyl and butyl, and can include a single alkyl or a plurality of alkyl bonded to one phenyl group. Typical hexahydrotriazine can be exemplified by 1,3,5-tribenzylhexahydrotriazine, 1,3,5-tris(3-methylbenzyl)hexahydrotriazine, 1,3,5-tris(2-hydroxyethyl)hexahydrotriazine, etc.

The thermal decomposition of hexahydrotriazine compound is endothermic, but, if the reaction temperature is too high, side reaction occurs together with the thermal decomposition reaction, thereby lowering the yield of methyleneimine compound, whereas, if too low, the thermal decomposition reaction hardly occurs. Thus, the reaction temperature is preferably 130° to 220°C, more preferably 160° to 190°C.

The present thermal decomposition reaction can be carried out in much better yield in the presence of a

catalyst of an amine salt, a quaternary ammonium salt or a mono- to tri-valent metal salt of organic acid or inorganic aicd.

The catalyst includes, for example, salts of organic acids such as formic acid, acetic acid, etc. or inorganic acids such as hydrochloric acid, hydrofluoric acid, hydrobromic acid, hydroiodic acid, sulfuric acid, etc. with amines such as benzylamine, metaxylylenediamine, etc., quaternary ammonium salts of these acids; iron chlorides (II or III), chromium chlorides (II or III), copper chlorides (I or II), copper fluorides (I or II), magnesium chloride, zinc chloride, etc.

It is preferable to use 1 to 100 m moles of the catalyst per mole of the hexahydrotriazine compound. If the amount of the catalyst is too small, no catalytic effect can be obtained, whereas, if too large, the effect has no significant difference, or rather fouling of reaction system or an increased load in washing operation may be brought about.

When the catalyst is used, the thermal decomposition reaction temperature can be made lower than that in the absence of the catalyst. In the presence of the catalyst, the reaction temperature is preferably 100° to 200°C, more preferably 100° to 160°C.

The presence of water in the reaction system promotes polymerization of methyleneimine compound, and thus it is preferable that the starting material hexahydrotriazine compound and the catalyst are free from water of

crystallization and absorbed water.

The methylenimine compound can be readily separated from the reaction product solution by distillation under reduced pressure. It is also possible to separate the methyleneimine compound by distillation while conducting the thermal decomposition reaction in a vacuum distillation apparatus.

The thus obtained methyleneimine compound is a compound having one methylene group at one tertiary nitrogen and is a colorless, transparent liquid of low viscosity at room temperature and having such a property that it can readily undergo polymerization reaction in the atmosphere to produce polymers, and thus can be used in various applications to an instantaneous adhesive or raw material for other chemical products as such, or to a catalyst for various urethane foams, a curing agent and a curing promoter for epoxy resin, a paint stabilizer, a curing agent for rubber latex, a vulcanization promoter, an electrode-protecting agent, or electro-conductive polymers to be obtained by quaternalizing the tertiary nitrogens in the polymers, ion exchange resins, polymeric coagulating agents, etc. as polymers. Furthermore, it can be used as a raw material for preparing other chemical products by utilizing the reactivity of tertiary amino group.

## PREFERRED EMBODIMENTS OF THE INVENTION

### Example 1

60 g of 1,3,5-tribenzylhexahydrotriazine was

charged into a two-necked, egg plant type flask with a thermometer in a vacuum distillation apparatus, and the atmosphere in the apparatus was replaced with dry nitrogen. Then, the flask was heated at a vacuum of 3 mmHg and 160°-180°C in a nitrogen gas stream to obtain benzylmethyleneimine as a distillate in a yield of 43 g, which corresponded to 71.7% of the theoretical yield on the basis of 1,3,5-tribenzylhexahydrotriazine.

Physical properties of the product are as follows:

Boiling point: 48°C/3 mmHg

Specific gravity (0°C): 0.994

Elemental analysis

| | Found | Calculated (as $C_8H_9N$) |
|---|---|---|
| C (%) | 80.60 | 80.67 |
| H (%) | 7.66 | 7.57 |
| N (%) | 11.72 | 11.76 |

Example 2

50 g (0.140 mole) of 1,3,5-tribenzylhexahydrotriazine and 0.107 g (0.754 m moles) of benzylamine hydrochloride were charged in the same apparatus as used in Example 1, and heated at a vacuum of 3 mmHg and 160°-170°C in a nitrogen gas stream, whereby 43 g of benzylmethyleneimine was obtained as a distillate.

Example 3

50 g (0.140 mole) of 1,3,5-tribenzylhexahydrotriazine and 0.818 g (3.48 m moles) of benzylamine hydroiodide were charged into the same apparatus as used in Example 1, and heated at a vacuum of 3 mmHg and 180°-185°C in a nitrogen gas stream, whereby 39 g of benzylmethyleneimine was obtained as a distillate.

Example 4

50 g (0.14 moles) of 1,3,5-tribenzylhexahydrotriazine and 0.140 g (1.41 m mole) of copper chloride (I) were charged into a two-necked, egg plant-type flask with a thermometer in a vacuum distillation apparatus, and the atmosphere in the apparatus was replaced with dry nitrogen. Then, the flask was heated at a vacuum of 3 mmHg and 130°-150°C in a nitrogen gas stream, whereby benzylmethyleneimine was obtained as a distillate in a yield of 47.8 g, which corresponded to 95.6% of the theoretical yield on the basis of 1,3,5-tribenzylhexahydrotriazine.

Example 5

50 g of 1,3,5-tris(3-methylbenzyl)hexahydrotriazine was charged into the same apparatus as used in Example 1, and heated at a vacuum of 1.5 mmHg and 190°-210°C in a nitrogen gas stream, whereby 3-methylbenzylmethyleneimine was obtained as a distillate in a yield of 35 g, which corresponded to 70% of the theoretical yield on the basis of 1,3,5-tris(3-methylbenzyl)hexahydrotriazine. The physical properties of the product are given below:

Boiling point: 67°C/1.5 mmHg

Specific gravity (°C): 0.966

Elemental analysis

| | Found | Calculated (as $C_9H_{11}N$) |
|---|---|---|
| C (%) | 81.32 | 81.20 |
| H (%) | 8.30 | 8.27 |
| N (%) | 10.45 | 10.53 |

Example 6

50 g (0.125 moles) of 1,3,5-tris(3-methylbenzyl)-hexahydrotriazine and 0.167 g (1.69 m moles) of copper chloride (I) were charged into the same apparatus as used in Example 1, and heated at a vacuum of 1.5 mmHg and 170°-190°C in a nitrogen gas stream, whereby 3-methylbenzylmethyleneimine was obtained as a distillate in a yield of 46.5 g, which corresponded to 93.0% of the theoretical value on the basis of 1,3,5-tris(3-methylbenzyl)hexahydrotriazine.

Example 7

50 g of 1,3,5-tris(2-hydroxyethyl)hexahydrotriazine was charged into the same apparatus as used in Example 1, and heated at a vacuum of 31 mmHg and 160°-190°C in a nitrogen gas stream, whereby 2-hydroxyethylmethylene-imine was obtained as a distillate in a yield of 32 g which corresponded to 64% of the theoretical yield on the basis of 1,3,5-tris(2-hydroxyethyl)hexahydrotriazine. The physical properties of the product are given below:

Boiling point: 77°-78°C/68 mmHg

Specific gravity (°C): 1.065

Elemental analysis

| | Found | Calculated (as $C_3H_7NO$) |
|---|---|---|
| C (%) | 49.22 | 49.32 |
| H (%) | 9.71 | 9.59 |
| N (%) | 19.33 | 19.18 |

Example 8

50 g (0.228 moles) of 1,3,5-tris(2-hydroxyethyl) hexahydrotriazine and 0.104 g (1.05 m moles) of copper chloride (I) were charged into the same apparatus as used in Example 1, and heated at a vacuum of 31 mmHg and 120°-140°C in a nitrogen gas stream, whereby 2-hydroxymethyleneimine was obtained as a distillate in a yield of 46 g, which corresponded to 92% of the theoretical yield on the basis of 1,3,5-tris(2-hydroxyethyl)hexahydrotriazine.

CLAIMS:

1. A process for producing a methyleneimine compound, represented by the general formula:

$$CH_2=N-CH_2-R$$

wherein R is phenyl, alkyl-substituted phenyl or hydroxymethyl, which comprises thermally decomposing a hexahydrotriazine compound represented by the general formula:

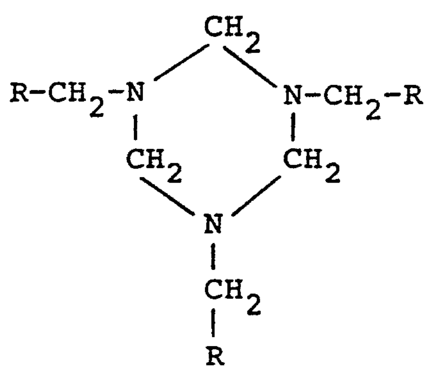

wherein R has the same meaning as defined above.

2. A process according to Claim 1, wherein the alkyl in the alkyl-substituted phenyl as R is methyl, ethyl, propyl or butyl and is bonded, singly or in a plurality thereof, to one phenyl group.

3. A process according to Claim 1, wherein the hexahydrotriazine compound is 1,3,5-tribenzylhexahydrotriazine, 1,3,5-tris(3-methylbenzyl)hexahydrotriazine, or 1,3,5-tris-(2-hydroxyethyl)hexahydrotriazine.

4. A process according to Claim 1, wherein the thermal decomposition is carried out at 130° to 220°C.

5. A process according to Claim 1, wherein the thermal decomposition is carried out in the presence of a catalyst of an amine salt, a quaternary ammonium salt, or at least one of a mono- to tri-valent metal salts of an organic acid or an inorganic acid.

6. A process according to Claim 5, wherein the catalyst is benzylamine, metaxylylenediamine, or quaternary ammonium salt of formic acid, acetic acid, hydrochloric acid, hydrofluoric acid, hydroiodic acid, or sulfuric acid, iron chloride (II or III), chromium chloride (II or III) copper chloride (I or II), copper fluoride (I or II), magnesium chloride or zinc chloride.

7. A process according to Claim 5, wherein 1 to 10 m mole of the catalyst is used per mole of the hexahydrotriazine compound.

8. A process according to Claim 5, wherein the thermal decomposition is carried out at 100° to 200°C.

9. A process according to Claim 1 or 5, wherein the hexahydrotriazine compound and the catalyst are free from water of crystallization and absorbed water.

European Patent Office

# EUROPEAN SEARCH REPORT

0149331

Application number

EP 84308595.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | US - A - 2 729 680 (J.L. ANDERSON) * Claims 1-4; column 5, line 4 - column 6, line 22 * | 1,3-9 | C 07 C 119/08 |
| X | JOURNAL OF THE CHEMICAL SOCIETY, 1953, London S.J. ANGYAL et al. "The Sommelet Reaction. Part VI. Methyleneamines. A Proposed Mechanism for the Reaction." pages 1742-1747 * Pages 1742-1744, 1746 * | 1-4,6, 8 | |
| | | | TECHNICAL FIELDS SEARCHED (Int. Cl.4) |
| | | | C 07 C 119/00 |

The present search report has been drawn up for all claims

| Place of search | Date of completion of the search | Examiner |
| --- | --- | --- |
| VIENNA | 26-03-1985 | HEIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82